# DEMANDE DE BREVET EUROPEEN

(11) **EP 1 900 352 A1**
(43) Date de publication de la demande: **19.03.2008**
(21) Numéro de dépôt: 07115010.6
(22) Date de dépôt: 27.08.2007
(51) Int. Cl.: A61K 8/37, A61K 8/72, A61K 8/73, A61Q 3/02

(54) **Vernis à ongles comprenant une résine**

(30) Priorité: 13.09.2006 FR 0653710
(71) Demandeur: L'Oréal, 75008 Paris (FR)
(72) Inventeur: COFFEY-DAWE, Lizabeth-Anne, 93600, AULNAY SOUS BOIS (FR); GUERCHET, Laurence, 94400, VITRY SUR SEINE (FR)
(74) Mandataire: Duvert, Sandra

(57) **Abrégé**

La présente invention a pour objet un vernis à ongles comprenant un milieu solvant organique, un agent gélifiant et au moins une résine cétone/aldéhyde. La présente invention a également pour objet un procédé cosmétique de maquillage ou de soin non thérapeutique des ongles comprenant l'application sur les ongles d'au moins une couche dudit vernis à ongles, ainsi que son utilisation pour obtenir, après dépôt sur l'ongle, un film qui est brillant.

## Description

La présente invention a pour objet une composition de vernis à ongles comprenant un milieu solvant organique et au moins une résine cétone/aldéhyde. Cette composition peut être appliquée sur les ongles d'êtres humains ou bien encore sur des faux ongles.

La composition de vernis à ongles, colorée ou transparente, peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles.

Il est courant d'utiliser, dans les compositions de vernis à ongles, des agents épaississants, en vue d'augmenter la viscosité de la phase organique de ces compositions. L'épaississement de la phase organique permet notamment d'améliorer la suspension des pigments présents dans cette phase et d'éviter la sédimentation de ceux-ci lors d'un stockage. L'épaississement de la phase organique facilite également la prise de la composition hors de son conditionnement et la répartition de ladite composition sur la zone à traiter.

Il est courant d'utiliser des agents épaississants, notamment des argiles telles que la bentone, en vue d'épaissir la phase organique des vernis à ongles. L'épaississement de la phase organique permet notamment d'améliorer la suspension des pigments présents dans cette phase et d'éviter la sédimentation de ceux-ci lors d'un stockage. L'épaississement de la phase organique facilite également la prise de la composition hors de son conditionnement et la répartition homogène de ladite composition sur l'ongle. Cependant, le fait que la bentone se présente sous forme de particules de tailles de l'ordre du micron, engendre une matification du film de vernis à ongle formé et une perte de brillance importance de ce film. On cherche donc à améliorer la brillance des compositions de vernis à ongles comprenant ce type d'agent épaississants.

Le but de la présente invention est donc de disposer de compositions de vernis à ongles présentant une brillance améliorée, tout en conservant une bonne stabilité dans le temps, et permettant la formation d'un film homogène sur les ongles.

Les inventeurs ont montré qu'il était possible d'augmenter la brillance des compositions de vernis à ongle en y introduisant au moins une résine spécifique.

La présente invention a donc pour objet un vernis à ongles comprenant un milieu solvant organique, au moins un agent gélifiant et au moins une résine cétone/aldéhyde.

L'invention a encore pour objet un procédé cosmétique de maquillage ou de soin non thérapeutique des ongles comprenant l'application sur les ongles d'au moins une couche d'un vernis à ongles telle que défini ci-dessus.

L'invention a également pour objet l'utilisation d'un un vernis à ongles comprenant un milieu solvant organique, au moins un agent gélifiant et au moins une résine cétone/aldéhyde, pour obtenir, après dépôt sur l'ongle, un film qui est brillant.

Le vernis à ongles selon l'invention comprend un milieu cosmétiquement acceptable, c'est-à-dire un milieu non toxique et susceptible d'être appliqué sur les matières kératiniques d'êtres humains, en particulier les ongles.

### Milieu solvant organique

La composition de vernis à ongle selon l'invention comprend un milieu solvant organique comprenant au moins un solvant organique ou un mélange de solvants organiques.

Le solvant organique peut être choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, l'acétate de t-butyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.

Le milieu solvant organique peut représenter de 10 à 95% en poids, par rapport au poids total de la composition, de préférence de 15% à 80% en poids, et mieux de 20 à 70% en poids.

### Milieu aqueux

Le milieu cosmétiquement acceptable de la composition selon l'invention peut également comprendre un milieu aqueux.
La teneur en milieu aqueux de la composition peut aller de 5 à 95 % en poids par rapport au poids total de la composition, de préférence de 50 à 70% en poids.
De préférence, la composition comprend moins de 10 % en poids d'eau, de préférence moins de 5% en poids par rapport au poids total de la composition.

### Résine cétone/aldéhyde

La composition de vernis à ongle selon la présente invention comprend au moins une résine cétone/aldéhyde.

De telles résines cétone/aldéhyde sont des produits de polycondensation (comprenant une étape de déshydratation) de mélanges d'au moins une cétone et d'au moins un aldéhyde.

Ces résines peuvent être préparées selon les procédés décrits dans les demandes de brevet US-A-2005/0080222 et US-A-2005/0124716 dont le contenu est introduit par référence dans la présente demande.

Les cétones pouvant être utilisées pour la préparation des résines selon l'invention peuvent être des cétones aliphatiques comprenant notamment de 1 à 20, et mieux de 1 à 10 atomes de carbone, et/ou des cétones cycloaliphatiques comprenant notamment de 5 à 20, et mieux de 6 à 12 atomes de carbone, seules ou en mélange. On peut notamment citer à titre d'exemple non limitatif l'acétone, l'acétophénone, la méthyle éthyle cétone, l'heptan-2-one, la méthyle isobutyle cétone, la cyclopentanone, la cyclododécanone, un mélange de 2,2,4 et de 2,4,4 triméthyle cyclohexanone, la cyclohexanone, un mélange de cycloheptanone et de cyclooctanone, et toutes les cyclohexanone substituées par au moins un groupement alkyle pouvant notamment comprendre entre 1 et 8 atomes de carbone. A titre d'exemple de cyclohexanone substituées par au moins un groupement alkyle, on peut citer la 4 ter-amylcyclohexanone, la 2 sec-butyle cyclohexanone, la 4 ter-butyle cyclohexanone, la 2 méthyle cyclohexanone, la 3,3,5 triméthyle cyclohexanone et leur mélange.

Selon un mode préféré de réalisation, les cétones préférées utilisées pour préparer les résines cétone/aldéhyde de la présente demande peuvent être des cétones cycloaliphatiques, notamment choisies parmi l'acétophénone, la cyclohexanone, la 4 ter-butyle cyclohexanone, la 3,3,5 triméthyle cyclohexanone, et leurs mélanges.

Selon un mode plus préféré de réalisation, la cétone est l'acétophénone.

Les aldéhydes pouvant être utilisés pour la préparation des résines cétone/aldéhyde selon l'invention peuvent être linéaires comprenant notamment de 1 à 20, et mieux de 1 à 15 atomes de carbone, ou ramifiés comprenant notamment de 5 à 20, mieux de 6 à 12 atomes de carbone, et leurs mélanges comme par exemple le formaldéhyde, l'acétaldéhyde, le n-butyraldéhyde et/ou l'iso-butyraldéhyde, le valeraldéhyde, le dodécanal et leurs mélanges.

Selon un mode préféré de réalisation, l'aldéhyde utilisé pour la synthèse des résines cétone/aldéhyde de l'invention peut être le formaldéhyde, seul ou en mélange avec d'autres aldéhydes.

Selon un mode plus préféré de réalisation, l'aldéhyde est le formaldéhyde.

Les aldéhydes utilisés peuvent se présenter sous forme de solution aqueuse ou alcoolique (par exemple en solution dans du méthanol ou du butanol), notamment a une concentration de 20 à 40% en poids.

Selon un mode particulier de réalisation, les résines cétone/aldéhyde sont des résines acétophénone/formaldéhyde.

Préférentiellement, les résines cétone/aldéhyde peuvent être introduites dans les compositions de vernis à ongle selon l'invention sous une forme hydrogénée ou sous une forme modifiée.

### Résines cétone/aldéhyde hydrogénées :

Les résines cétone/aldéhyde peuvent notamment être hydrogénées en présence d'un catalyseur approprié et à une pression pouvant aller jusqu'à 300 bar. Au cours de cette étape d'hydrogénation, le groupement carbonyle de la résine cétone/aldéhyde est transformé en groupe hydroxyle.

Selon un mode préféré de réalisation, les résines cétone/aldéhyde peuvent être des résines acétophénone/formaldéhyde hydrogénées obtenues comme suit :

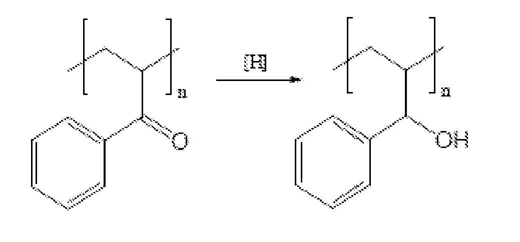

### Résines cétone/aldéhyde modifiées :

Les résines cétone/aldéhyde modifiées peuvent notamment être obtenues par réaction d'au moins une résine cétone/aldéhyde hydrogénée telle que celles décrites précédemment, et d'au moins un composé apte à réagir avec ladite résine cétone/aldéhyde hydrogénée.

A titre d'exemple, le composé apte à réagir avec la résine cétone/aldéhyde hydrogénée peut être choisi parmi les anhydrides maléiques, les dérivés d'acide (méth)acrylique, les isocyanates, notamment les diisocyanates, ou les polyisocyanates comprenant plus de deux groupement isocyanate par molécule.

Les diisocyanates pouvant être utilisés pour modifier la résine cétone/aldéhyde hydrogénée peuvent être choisis parmi les diisocyanates d'alkyle linéaire ou ramifié en C₁-C₁₂, de préférence en C₁-C₆, les diisocyanates de cycloalkyle en C₄-C₂₀ , les diisocyanates d'aryle en C₆-C₂₀. Ces diisocyanates peuvent notamment être choisis parmi le l'hexaméthylène diisocyanate (HDI), cyclohexyle diisocyanate, le méthyle cyclohexyle diisocyanate, l'éthyle cyclohexyle diisocyanate, le propyle cyclohexyle diisocyanate, le méthyle diéthyle cyclohexyle diisocyanate, le phényle diisocyanate, le tolylène diisocyanate, le bis(isocyanatophényl)méthane, le propane diisocyanate, le butane diisocyanate, le pentane diisocyanate, l'hexane diisocyanate, l'heptane diisocyanate, l'octane diisocyanate, le nonane diisocyanate, l'isophorone diisocyanate (IPDI), bis(isocyanatométyle cyclohexyl)méthane (H₁₂MDI), l'isocyanato méthyle méthyle cyclohexyle isocyanate, ou leurs mélanges.

Selon un mode préféré de réalisation, le composé utilisé pour modifier la résine cétone/aldéhyde hydrogénée peut être un diisocyanate, et en particulier l'isophorone diisocyanate (IPDI).

Selon un mode particulièrement préféré de réalisation, la résine cétone/aldéhyde utilisée dans les compositions de l'invention est une résine acétophénone/formaldéhyde hydrogénée modifiée par l'isophorone diisocyanate (IPDI) obtenue comme suit :

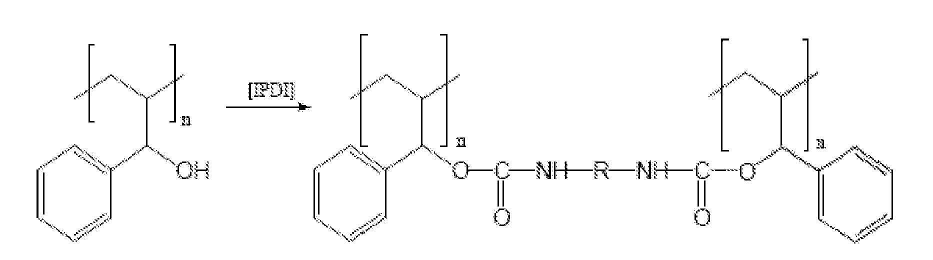

La résine cétone/aldéhyde pouvant être utilisée dans les compositions selon l'invention peut notamment être choisie parmi les résines commercialisées sous les dénomination Synthetic Resin SK, ou Synthetic Resin 1201 par la société DEGUSSA.

La résine cétone/aldéhyde peut être introduite dans les compositions selon l'invention en une teneur allant de 0,01 à 20% en poids en matière active, par rapport au poids total de la composition, de préférence de 0,05 à 15% en poids, et plus préférentiellement de 0,1 à 8% en poids.

### Polymère filmogène

Outre la résine cétone/aldéhyde précédemment décrite, la composition selon l'invention peut comprendre avantageusement un polymère filmogène.

On entend par "polymère filmogène" au sens de la présente invention, un polymère apte à former à lui seul ou en présence d'un agent auxiliaire de filmification, un film continu sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines arylsulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résines toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences du polymère séquencé sont incompatibles l'une avec l'autre.
De tels polymères sont décrits par exemple dans les documents EP 1411069 ou WO04/028488.

Le polymère filmogène additionnel peut être présent dans la composition selon l'invention en une teneur allant de 0,1 % à 60 % en poids, par rapport au poids total de la composition, de préférence allant de 2 % à 40 % en poids, et mieux de 5 % à 25 % en poids.

### Agent auxiliaire de filmification

Pour améliorer les propriétés filmogènes de la composition de vernis à ongles un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).
Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- des dérivés oxyéthylénés, tels que les huiles oxyéthylénées, notamment les huiles végétales, telles que l'huile de ricin ; et
- leurs mélanges.

Le type et la quantité d'agent plastifiant et/ou de coalescence peuvent être choisis par l'homme du métier sur la base de ses connaissances générales.
Par exemple, la teneur en agent plastifiant et/ou de coalescence peut aller de 0,01 % à 20% et en particulier de 0,5 % à 10 % en poids par rapport au poids total de la composition.

### Agent gélifiant

La composition comprend au moins un agent gélifiant (ou épaississant).

La proportion totale en agent(s) épaississant(s) dans les compositions selon l'invention peut aller de à 0,01 à 15 % en poids, par rapport au poids total de la composition, de préférence de 0,5 à 15% et mieux de 0,5 à 10% en poids.

Cet agent gélifiant peut en particulier être choisi parmi : les silices hydrophobes, telles que celles décrites dans le document EP-A-898960, et par exemple commercialisées sous les références "AEROSIL R812 par la société Degussa, "CAB-O-SIL TS-530^{®}", "CAB-O-SIL TS-610^{®}", "CAB-O-SIL TS-720 "par la société Cabot, "AEROSIL R972^{®}", "AEROSIL R974 " par la société Degussa ; les argiles organophiles telles que la montmorillonite, les argiles modifiées telles que les bentones par exemple, l'hectorite stéaralkonium, la bentonite stéaralkonium , les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

En particulier, l'agent gélifiant comprend au moins une argile organophile.
Les argiles organophiles sont des argiles modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Elementis, Tixogel VP par la société United catalyst, Claytone 34, Claytone 40, Claytone XL par la société Southern Clay; les stéaralkonium bentonites telles que celles vendues sous les dénominations Bentone 27V par la société Elementis, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.
L'argile organophile est en particulier choisie parmi les hectorites modifiées telles que l'hectorite modifiée par le stéarate de benzyl di methyl ammonium.

L'argile organophile peut être présent en une teneur supérieure ou égale à 0,5% en poids, allant par exemple de 0,5 % à 10 % en poids, par rapport au poids total de la composition, de préférence supérieure ou égale à 0,7% en poids, allant par exemple de 0,7 % à 5% en poids.

### Matière colorante

La composition selon l'invention peut, en outre, comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

L'invention est illustrée plus en détail dans les exemples suivants. Sauf indication contraire, les quantités sont données en pourcentage en poids par rapport au poids total de la composition.

### Exemple 1 : Vernis à ongles

On a préparé deux vernis à ongles ayant les compositions suivantes (% en poids) :

| | **Composition 1 (invention)** | **Exemple comparatif 1 (hors invention)** |
|---|---|---|
| Nitrocellulose à 30% d'alcool isopropylique (viscosité: E22 - 1 /2 S) | 11,49 | 12,96 |
| Alcool isopropylique pur | 3,67 | 3,67 |
| Nitrocellulose à 30 % d'alcool isopropylique (Azur E80 de Bergerac) | 1,18 | 1,44 |
| Résine cétone/formaldéhyde hydrogénée(¹) | 5,00 | 0,00 |
| Résine éthyl tosylamide | 2,92 | 2,92 |
| Résine alkyde glycérophtalique ésterifiée par acides gras ramifiés dans l'acétate d'éthyle à 70% (Beckosol ODE 230 70E de Dainippon Ink and Chemicals). | 16,17 | 16,17 |
| Hectorite modifiée stéaryl benzyl diméthyl ammonium (BENTONE 27 V d'Elementis) | 1,31 | 1,31 |
| Laque rouge vendue sous la dénomination D&C red 7 CA lake C 19-011 par la société SUN | 0,21 | 0,21 |
| Oxyde de titane enrobé polyéthylène oxydé | 0,02 | 0,02 |
| Laque bordeaux vendue sous la dénomination D&C red 34 CA lake C 24-012 par la société SUN | 0,56 | 0,56 |
| Acétate d'éthyle | 20,75 | 22,97 |
| Acétyl citrate de tributyle | 3,95 | 3,95 |
| Acétate de butyle | 32,72 | 33,77 |
| Acide citrique, 1 H2O | 0,05 | 0,05 |

| | | |
|---|---|---|
| ⁽¹⁾ vendue sous la dénomination Synthetic resin SK par la société DEGUSSA. | | |

### On a mesuré la brillance de chaque vernis selon le protocole suivant :

Sur une carte de contraste de marque LENETA et de référence FORM 1A PENOPAC, on étale une couche de 300 µm d'épaisseur humide de la composition dont on cherche à évaluer la brillance moyenne, à l'aide d'un étaleur automatique. La couche recouvre le fond blanc et la fond noir de la carte. On laisse sécher pendant 24 heures sur un banc thermostaté à 30°C puis on procède à la mesure de la brillance à 20° et à 60° sur le fond blanc (3 mesures) et sur le fond noir (3 mesures) à l'aide d'un brillancemètre de marque BYK GARDNER et de référence microTRI-GLOSS.

L'indice de flou (ou Haze) permet de quantifier le ternissement du film de vernis à ongles.
On l'a mesuré selon le protocole suivant :
Sur une carte de contraste de marque LENETA et de référence FORM 1A PENOPAC, on étale une couche de 300 µm d'épaisseur humide de la composition dont on cherche à évaluer la brillance moyenne, à l'aide d'un étaleur automatique. La couche recouvre le fond blanc et le fond noir de la carte. On laisse sécher pendant 24 heures sur un banc thermostaté à 30°C puis on procède à la mesure de l'indice de flou (ou Haze) sur le fond noir à l'aide d'un brillancemètre/hazemètre de marque BYK GARDNER et de référence Haze Gloss. On reproduit cette mesure à 5 reprises, la valeur de l'indice de flou obtenue correspond à la moyenne calculée des 5 valeurs mesurées.

On obtient les résultats suivants :

| | Composition 1 (invention) | Exemple comparatif 1 (hors invention) |
|---|---|---|
| Brillance (20°) | 76,1 | 66,8 |
| Haze | 132 | 188 |

### Exemple 2 : Vernis à ongles

On a préparé deux vernis à ongles ayant les compositions suivantes (% en poids) :

| | **Composition 2 (invention)** | **Exemple comparatif 2 (hors invention)** |
|---|---|---|
| Nitrocellulose à 30% d'alcool isopropylique (viscosité: E22 - 1 /2 S) | 11,34 | 12,81 |
| Alcool isopropylique pur | 3,70 | 3,70 |
| Nitrocellulose à 30 % d'alcool isopropylique (Azur E80 de Bergerac) | 1,40 | 1,66 |
| Résine cétone/formaldéhyde hydrogénée(¹) | 5,00 | 0 |
| Résine éthyl tosylamide | 4,77 | 4,77 |
| Résine alkyde glycérophtalique ésterifiée par acides gras ramifiés dans l'acétate d'éthyle à 70% (Beckosol ODE 230 70E de Dainippon | 16, 07 | 16, 07 |
| Ink and Chemicals). | | |
| Hectorite modifiée stéaryl benzyl diméthyl ammonium (BENTONE 27 V d'Elementis) | 1,28 | 1,28 |
| Pigment bleu ferrique | 0,0003 | 0,0003 |
| Oxyde de fer brun enrobé de polyéthylène oxydé | 0,03 | 0,03 |
| Oxyde de titane enrobé polyéthylène oxydé | 0,54 | 0,54 |
| Laque jaune vendue sous la dénomination D&C Yel 5 AL lake C 69-4424 par la société SUN | 0,02 | 0,02 |
| Acétate d'éthyle | 21,03 | 23,24 |
| Acétyl citrate de tributyle | 2,06 | 2,06 |
| Acétate de butyle | 32,71 | 33,76 |
| Acide citrique, 1 H2O | 0,05 | 0,05 |

| | | |
|---|---|---|
| ⁽¹⁾ vendue sous la dénomination Synthetic resin SK par la société DEGUSSA. | | |

On a mesuré la brillance et l'indice de flou (ou Haze) de chaque vernis selon le protocole décrit dans l'exemple 1.

On obtient les résultats suivants :

| | Composition 2 (invention) | Exemple comparatif 2 (hors invention) |
|---|---|---|
| Brillance (20°) | 78 | 61,6 |
| Haze | 140 | 191 |

### Exemple 3 : Vernis à ongles

On a préparé deux vernis à ongles ayant les compositions suivantes (% en poids) :

| | **Composition 3 (invention)** | **Exemple comparatif 3 (hors invention)** |
|---|---|---|
| Nitrocellulose à 30% d'alcool isopropylique (viscosité: E22 - 1 /2 S) | 8,40 | 10,48 |
| Alcool isopropylique pur | 4,74 | 4,02 |
| Nitrocellulose à 30 % d'alcool isopropylique | 1,17 | 2,56 |
| (Azur E80 de Bergerac) | | |
| Copolymère de méthacrylate de méthyle/méthacrylate de butyle | 1,00 | 1,00 |
| Résine cétone/formaldéhyde hydrogénée (¹) | 5,00 | 0 |
| Résine éthyl tosylamide | 2,04 | 2,04 |
| Résine alkyde glycérophtalique ésterifiée par acides gras ramifiés dans l'acétate d'éthyle à 70% (Beckosol ODE 230 70E de Dainippon Ink and Chemicals). | 6,87 | 6,87 |
| Hectorite modifiée stéaryl benzyl diméthyl ammonium (BENTONE 27 V d'Elementis) | 1,27 | 1,27 |
| Laque rouge vendue sous la dénomination D&C red 7 CA lake C 19-011 par la société SUN | 0,21 | 0,21 |
| Oxyde de titane enrobé polyéthylène oxydé | 0,02 | 0,02 |
| Laque bordeaux vendue sous la dénomination D&C red 34 CA lake C 24-012 par la société SUN | 0,56 | 0,56 |
| Acétate d'éthyle | 33,14 | 31,09 |
| Acétyl citrate de tributyle | 5,47 | 5,47 |
| Acétate de butyle | 30,06 | 34,36 |
| Acide citrique, 1 H2O | 0,05 | 0,05 |

| | | |
|---|---|---|
| ⁽¹⁾ vendue sous la dénomination Synthetic resin SK par la société DEGUSSA. | | |

On a mesuré la brillance et l'indice de flou (ou Haze) de chaque vernis selon le protocole décrit dans l'exemple 1.

On obtient les résultats suivants :

| | Composition 3 (invention) | Exemple comparatif 3 (hors invention) |
|---|---|---|
| Brillance (20°) | 64,5 | 48,6 |
| Haze | 187 | 249 |

## Revendications

1. Vernis à ongles comprenant un milieu solvant organique **caractérisé en ce qu'**il comprend au moins une résine cétone/aldéhyde et au moins un agent gélifiant.

2. Vernis à ongles selon la revendication précédente, **caractérisé en ce que** le milieu solvant organique comprend au moins un solvant organique choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, l'acétate de t-butyle, le lactate de butyle et leurs mélanges.

3. Vernis à ongles selon l'une quelconque des revendications précédentes, **caractérisée en ce que** le milieu solvant organique représente de 10 à 95% en poids, par rapport au poids total de la composition, de préférence de 15% à 80% en poids, et mieux de 20 à 70% en poids.

4. Vernis à ongles selon l'une quelconque des revendications précédentes, **caractérisée en ce que** la résine cétone/aldéhyde est un produit de polycondensation de mélange d'au moins une cétone et d'au moins un aldéhyde.

5. Vernis à ongles selon la revendication précédente, **caractérisée en ce que** la cétone est choisie parmi les cétones aliphatiques comprenant notamment de 1 à 20, et mieux de 1 à 10 atomes de carbone, et/ou des cétones cycloaliphatiques comprenant notamment de 5 à 20, et mieux de 6 à 12 atomes de carbone, seules ou en mélange.

6. Vernis à ongles selon la revendication précédente, **caractérisée en ce que** la cétone est une cétone cycloaliphatique, notamment choisie parmi choisie parmi l'acétone, l'acétophénone, la méthyle éthyle cétone, l'heptan-2-one, la méthyle isobutyle cétone, la cyclopentanone, la cyclododécanone, un mélange de 2,2,4 et de 2,4,4 triméthyle cyclohexanone, la cyclohexanone, un mélange de cycloheptanone et de cyclooctanone, et toutes les cyclohexanone substituées par au moins un groupement alkyle pouvant notamment comprendre entre 1 et 8 atomes de carbone telles que la 4 ter-amylcyclohexanone, la 2 sec-butyle cyclohexanone, la 4 ter-butyle cyclohexanone, la 2 méthyle cyclohexanone, la 3,3,5 triméthyle cyclohexanone et leur mélange.

7. Vernis à ongles selon la revendication précédente, **caractérisée en ce que** la cétone est l'acétophénone.

8. Vernis à ongles selon la revendication 4, **caractérisé en ce que** l'aldéhyde est choisi parmi les aldéhydes linéaires comprenant notamment de 1 à 20, et mieux de 1 à 15 atomes de carbone, ou ramifiés comprenant notamment de 5 à 20, mieux de 6 à 12 atomes de carbone, et leurs mélanges.

9. Vernis à ongles selon l'une des revendications 4 et 8, **caractérisé en ce que** l'aldéhyde est choisi parmi le formaldéhyde, l'acétaldéhyde, le n-butyraldéhyde et/ou l'iso-butyraldéhyde, le valeraldéhyde, et le dodécanal.

10. Vernis à ongles selon l'une des revendications 4, 8 et 9, **caractérisé en ce que** l'aldéhyde est le formaldéhyde.

11. Vernis à ongles selon l'une quelconque des revendications précédentes, **caractérisé en ce que** la résine est une résine acétophénone/formaldéhyde.

12. Vernis à ongles selon l'une quelconque des revendications précédentes, **caractérisé en ce que** la résine cétone/aldéhyde est une résine acétophénone/formaldéhyde hydrogénée.

13. Vernis à ongles selon l'une quelconque des revendications précédentes, **caractérisé en ce que** la résine est une résine acétophénone/formaldéhyde hydrogénée modifiée.

14. Vernis à ongles selon la revendication précédente, **caractérisée en ce que** la résine acétophénone/formaldéhyde hydrogénée modifiée est obtenue par réaction d'une résine acétophénone/formaldéhyde hydrogénée avec un isocyanate, notamment un diisocyanate.

15. Vernis à ongles selon la revendication précédente, **caractérisée en ce que** le diisocyanate est choisi parmi les diisocyanates d'alkyle linéaire ou ramifié en C₁-C₁₂, de préférence en C₁-C₆, les diisocyanates de cycloalkyle en C₄-C₂₀ , les diisocyanates d'aryle en C₆-C₂₀.

16. Vernis à ongles selon l'une quelconque des revendications 13 à 15, **caractérisée en ce que** la résine acétophénone/formaldéhyde hydrogénée modifiée est obtenue par réaction d'une résine acétophénone/formaldéhyde hydrogénée avec l'isophorone diisocyanate (IPDI).

17. Vernis à ongles selon l'une quelconque des revendications précédentes, **caractérisé en ce que** la résine cétone/aldéhyde est présente en une teneur en matière active allant de 0,01 à 20% en poids en matière active, par rapport au poids total de la composition, de préférence de 0,05 à 15% en poids, et plus préférentiellement de 0,1 à 8% en poids.

18. Vernis à ongles selon l'une quelconque des revendications précédentes, **caractérisé en ce qu'**il comprend au moins un polymère filmogène.

19. Vernis à ongles selon la revendication précédente, **caractérisé en ce que** le polymère filmogène est présent en une teneur allant de 0,1 % à 60 % en poids, par rapport au poids total de la composition, de préférence allant de 2 % à 40 % en poids, et mieux de 5 % à 25 % en poids.

20. Vernis à ongles selon l'une quelconque des revendications précédentes, **caractérisé en ce qu'**il comprend au moins un agent auxiliaire de filmification.

21. Vernis à ongles selon l'une quelconque des revendications précédentes, **caractérisé en ce que** l'agent gélifiant comprend au moins une argile organophile.

22. Vernis à ongles selon la revendications précédente, **caractérisé en ce que** l'argile organophile est choisie parmi les hectorites modifiées telles que l'hectorite modifiée par le stéarate de benzyl di methyl ammonium .

23. Vernis à ongles selon la revendication 21 ou 22, **caractérisé en ce que** l'argile organophile est présente en une teneur supérieure ou égale à 0,5% en poids, allant par exemple de 0,5 % à 10 % en poids, par rapport au poids total de la composition, de préférence supérieure ou égale à 0,7% en poids, allant par exemple de 0,7 % à 5% en poids.

24. Vernis à ongles selon l'une quelconque des revendications précédentes, **caractérisé en ce qu'**il comprend au moins une matière colorante.

25. Vernis à ongles selon la revendication 24, **caractérisé en ce que** la matière colorante est présente en une teneur allant de 0,01 % à 50 % en poids, par rapport au poids de la composition, de préférence de 0,01 % à 30 % en poids.

26. Procédé cosmétique de maquillage ou de soin non thérapeutique des ongles comprenant l'application sur les ongles d'au moins une couche d'un vernis à ongles selon l'une des revendications 1 à 25.

27. Utilisation d'un vernis à ongles selon l'une quelconque des revendications 1 à 25 pour obtenir, après dépôt sur l'ongle, un film qui est brillant.
